# EUROPÄISCHE PATENTANMELDUNG

(11) **EP 0 672 428 A2**
(43) Veröffentlichungstag der Anmeldung: **20.09.1995**
(21) Anmeldenummer: 95102350.6
(22) Anmeldetag: 20.02.1995
(51) Int. Cl.: A61M 5/32

(54) **Sicherheitsbehälter zum Sammeln scharfer oder spitzer Gegenstände**

(30) Priorität: 21.02.1994 DE 4405483
(71) Anmelder: Uson, Josef, D-80799 München (DE)
(72) Erfinder: Uson, Josef, D-80799 München (DE)
(74) Vertreter: Fincke, Karl Theodor, Dipl.-Phys. Dr.

(57) **Zusammenfassung**

Es werden Sicherheitsbehälter (8) beschrieben, und zwar zum Vernichten scharfer oder spitzer Gegenstände, insbesondere im klinischen Bereich von Kanülen und Klingen, die an einem Halter, insbesondere einem Spritzenkörper oder an einem Skalpellgriff, befestigt sind, mit einem durch eine Wand (10) ummantelten Innenbereich, einer mit der Wand (10) abschließenden, geschlossenen Bodenfläche (12), und einer mit der Wand (10) abschließenden Deckfläche (16), die gegenüberliegend zur Bodenfläche (12) angeordnet ist, wobei die Deckfläche (16) eine Öffnung (18) aufweist, durch welche die zu vernichtenden scharfen oder spitzen Gegenstände in den Innenbereich einführbar sind, und bei dem eine Entfernvorrichtung (14) vorgesehen ist, mit der spitze oder scharfe Gegenstände vom Halter einhändig entfernt werden können, wobei die Sicherheitsbehälter (8) dadurch gekennzeichnet sind, daß die Öffnung (18) in jeder Richtung kleiner als die jeweils größte Abmessung des kleinsten zu vernichtenden Gegenstandes ist, jedoch so groß ist, daß die Gegenstände innerhalb eines vorgegebenen Winkelbereichs durch die Öffnung (18) geführt werden können, daß der Winkelbereich so vorgegeben ist und die Form von Deckfläche (16) und Wand (10) sowie die Lage der Öffnung (18) in der Deckfläche (16) so gewählt sind, daß die Gegenstände nicht durch die Öffnung (18) gleiten können, wenn der Sicherheitsbehälter (8) stabil auf Teilen der Wand (10) oder der Bodenfläche (12) aufstößt oder ruht, und daß eine mit der Deckfläche (16) des Sicherheitsbehälters (8) fest verbundene Führungseinrichtung (16) zur Führung und Ausrichtung der durch die Entfernungsvorrichtung (14) vom Halter entfernten Gegenstände vorgesehen ist oder die Deckfläche(16) zumindest teilweise als Führungseinrichtung ausgebildet ist, wobei die Führungseinrichtung (16) sich mindestens von der Entfernvorrichtung (14)zu der Öffnung (18) erstreckt und geeignet ist, die Gegenstände innerhalb des Winkelbereichs auszurichten und durch die Öffnung (18) zu führen. Weiter werden damit zusammenhängende Arbeitsverfahren und klinische Systeme gelehrt.

## Beschreibung

Die Erfindung betrifft Sicherheitsbehälter zum Vernichten scharfer oder spitzer Gegenstände, insbesondere im klinischen Bereich von Kanülen und Klingen, die an einem Halter, insbesondere an einem Spritzenkörper oder einem Skalpellgriff, befestigt sind, mit einem durch eine Wand ummantelten Innenbereich, einer mit der Wand abschließenden, geschlossenen Bodenfläche, und einer mit der Wand abschließenden Deckfläche, die gegenüberliegend zur Bodenfläche angeordnet ist, wobei die Deckfläche eine Öffnung aufweist, durch welche die zu vernichtenden scharfen oder spitzen Gegenstände in den Innenbereich einführbar sind, und bei dem eine Entfernvorrichtung, vorgesehen ist, mit der spitze oder scharfe Gegenstände von dem Halter einhändig entfernt werden können. Weiter wird ein Arbeitsverfahren zum Arbeitsschutz im klinischen Bereich gelehrt, welches einen Schritt zur Behandlung eines Patienten an einem Arbeitsplatz an dem spitze oder scharfe, an einem Halter angeordnete Gegenstände verwendet werden, enthält. Ferner betrifft die Erfindung ein klinisches System zur Behandlung von Patienten mit Arbeitsplätzen, an denen Arbeitsgerätschaften mit spitzen oder scharfen Gegenständen, wie die Kanülen von Spritzen oder die Klingen von Skalpellen verwendet werden.

In Krankenhäusern, Arztpraxen, Altenheimen u. ä. besteht eine große Verletzungsgefahr durch spitze und scharfe Gegenstände, wie Spritzen, Nadeln und Skalpeile. Besonders gefährlich ist eine Verletzung dann, wenn diese Gegenstände schon am Patienten verwendet wurden, da dann neben einer Verwundung auch eine erhöhte Infektionsgefahr auftreten kann.

So wird in einem Bericht des Bundesverbands der Unfallversicherer BAGUV vom Dezember 1990 über "Unfälle in Krankenhäusern 1980-1989" angegeben, daß 48% aller Arbeitsunfälle auf Verwundungen mit Spritzen. Kanülen und Skalpelle entfallen. Bei zehntausend Unfällen im Jahr im Klinikbereich ist das eine große, auf die Dauer nicht vertretbare Zahl. Ähnliche dramatische Zahlen gehen auch aus Untersuchungen aus dem Jahre 1984 und 1985 am Universitätsklinikum Freiburg hervor. Die Berufsgenossenschaft für Gesundheits- und Wohlfahrtspflege berichtet von ungefähr 700 Unfallmeldungen pro Jahr, bei denen Arbeitsunfähigkeit von mehr als 3 Tagen vorliegt.

Nicht nur für die im Krankenhaus arbeitenden Menschen ist ein verbesserter Arbeitsschutz zur Erhaltung der Gesundheit notwendig, sondern er kann auch zur Verringerung der Kosten im Gesundheitswesen, beitragen. Die genannten 700 Unfälle im Jahr nur einer einzigen Berufsgenossenschaft ergeben allein bei 3 Tagen, wenn man die Kosten für einen Tag mit 200,-DM ansetzt, schon einen Betrag von über vierhunderttausend Mark. Berücksichtigt man weiter, daß dies nur die Zahlen einer einzigen Berufsgenossenschaft sind und eine Arbeitsunfähigkeit oft mehr als drei Tage dauern kann, muß man mit einen volkswirtschaftlichen Schaden aufgrund dieser Unfälle von mehreren Millionen rechnen.

Vor allem da gefährliche, bei Verletzungen leicht übertragbare Krankheiten wie Aids zunehmen ist eine Verringerung von Verletzungen mit infizierten Spritzen, Nadeln und Skalpellen dringend erforderlich.

Eine einfache Möglichkeit zur Vermeidung von Verletzungen nach Gebrauch, bestände darin, einen verschließbaren Sicherheitsbehälter vorzusehen, in den die Nadeln, Skalpelle oder Spritzen nach Gebrauch geworfen werden. Das Problem verschiebt sich dann vom Klinikbereich auf das mit der Entsorgung befaßte Personal, denn wenn die Spritzen dem Sicherheitsbehälter entnommen werden, können ebenfalls Verletzungen entstehen.

Um die Infektionsgefahr zu vermindern , müssen Spritzen und Skalpelle jetzt schon als Sondemüll entsorgt werden (siehe beispielsweise Abfallsatzung vom 17.7.92 der Landeshauptstadt München, Amtsblatt 21/1992, §3 und deren Anlage zu Abs. 1, Nr.18: krankenhausspezifische Abfälle, Abfallschlüssel Nr. 97,971 und 97101). Deswegen erhöht das Wegwerfen ganzer Spritzen oder Skalpelle auch den Sondermüllanfall, so daß hier neue Kosten entstehen. Es ist auch zu beachten, daß ganze Spritzen oder Kanülen sehr sperrig sind, so daß nur ein Teilbereich des Sicherheitsbehälters gefüllt wird, was das Müllvolumen weiter erhöht. Die Probleme der Müllentsorgung sind allgemein bekannt.

Es gibt Spritzen mit abnehmbaren Kanülen, das Abnehmen der Kanülen erhöht aber die Verletzungsgefahr. Zur Vermeidung solcher Verletzungen können Vorrichtungen eingesetzt werden, wie sie aus der Patentliteratur bekannt sind.

Die DE 33 30 92 lehrt einen Aufbau für einen medizinischen Arbeitsplatz mit einem unter einer Entfernvonrichtung für das einhändige Entfernen der Kanülen von Spritzen stehenden Sicherheitsbehälter, in den die Kanülen zur späteren Entsorgung aufbewahrt werden. Nachteilig dabei ist, daß an jedem Arbeitsplatz eine entsprechend aufgebaute Entfernvorrichtung angeordnet werden muß, was Investitionen erfordert. Weiter kann sich der Sicherheitsbehälter beispielsweise beim unbeabsichtigten Umstoßen leicht entleeren, was immer noch ein erhöhtes Verletzungs- und Infektionsrisiko bedeutet.

Bei Sicherheitsbehältern gemäß der DE 40 130 13 A1 oder der DE 35 01 043 lassen sich Kanülen direkt an einer an einem Sicherheitsbehälter vorgesehenen Entfernvorrichtung von dem Spritzenkörper abstreifen. Nach Abstreifen fällt die Kanüle in einen Sicherheitsbehälter, dessen Öffnung automatisch verschlossen wird, so daß auch ein unbeabsichtigtes Umstoßen des Sicherheitsbehälters keine Gefahr darstellt. Derartige Vorrichtungen bedürfen aber mechanisch beweglicher Teile, was aufwendig ist und Kosten verursacht, weswegen sie möglicherweise für eine Ausstattung aller medizinischer Arbeitsplätze ungeeignet sind.

Außerdem lassen sich die angegebenen Sicherheitsbehältern auch nicht für Skalpelle verwenden. Wünschenswert ist daher ein Sicherheitsbehälter der genannten Art, der sich kostengünstig herstellen läßt, indem er keine beweglichen Teile verwendet, einen Großteil von möglichen Verletzungen bei unbeabsichtigtem Umstoßen vermeidet und der sich zur Entsorgung möglichst vieler Arten von spitzen und scharfen Gegenständen eignet.

Aufgabe der Erfindung ist es, einen zum genannten Stand der Technik alternativen Sicherheitsbehälter zu schaffen, mit dem bei einem entsprechenden Arbeitsverfahren in einem klinischen System die höchstmögliche Sicherheit bei verringerten Kosten erreicht wird.

Die Aufgabe wird erfindungsgemäß durch einen Sicherheitsbehälter der eingangs genannten Art gelöst, bei dem die Öffnung in jeder Richtung kleiner als die jeweils größte Abmessung des kleinsten zu vernichtenden Gegenstandes ist, jedoch so groß ist, daß die Gegenstände innerhalb eines vorgegebenen Winkelbereichs durch die Öffnung geführt werden können, bei dem der Winkelbereich so vorgegeben ist und die Form von Deckfläche und Wand sowie die Lage der Öffnung in der Deckfläche so gewählt sind, daß Gegenstände nicht durch die Öffnung gleiten können, wenn der Sicherheitsbehälter stabil auf Teilen der Wand oder der Bodenfläche aufstößt oder ruht, und bei dem eine mit der Deckfläche des Sicherheitsbehälters fest verbundene Führungseinrichtung zur Führung und Ausrichtung der durch die Entfernungsvorrichtung vom Halter entfernten Gegenstände vorgesehen ist oder bei dem die Deckfläche zumindest teilweise als Führungseinrichtung ausgebildet ist, wobei die Führungseinrichtung sich mindestens von der Entfernvorrichtung zu der Öffnung erstreckt und geeignet ist, die Gegenstände innerhalb des Winkelbereichs auszurichten und durch die Öffnung zu führen.

Bei einem erfindungsgemäßer Sicherheitsbehälter können also Kanülen oder andere scharfe oder spitzen Gegenstände nur innerhalb eines definierten Winkelbereichs durch die Öffnung geführt werden. Dazu dient eine Führungseinrichtung, welche die Gegenstände von der Entfernvorrichtung zu der Öffnung gleiten läßt und ausrichtet. Das hat unter anderem gegenüber dem Stand der Technik den Vorteil, daß über mehrere Führungsvorrichtungen an verschiedenen Stellen der Öffnung oder mehrere Öffnungen spitze oder scharfe Gegenstände in den Innenbereich des Sicherheitsbehälters eingebracht werden können. Die Erfindung ermöglicht also die Verwendung verschiedener Entfernvorrichtungen für verschiedenste Gegenstände, beispielsweise Kanülen unterschiedlicher Abmessungen, Skalpelle oder auch Abbrechvorrichtungen zur Beseitigung der Nadeln von Einmalspritzen, um den Sondermüll zu verringern.

Man könnte allerdings daran denken, eine Deckfläche so auszubilden, daß keine Führungseinrichtung nötig ist, indem eine Deckfläche eines Sicherheitsbehälters mit einer Öffnung versehen wird, mit einer Form, die beispielsweise das Abstreifen einer Kanüle von einer Spritze erlaubt. Dann würde aber im allgemeinen die Öffnung größer als notwendig ausgeführt werden müssen, damit der Arbeitsgang nicht durch eine sorgfältige Ausrichtung der Spritze erschwert wird. Diese Vergrößerung der Öffnung würde jedoch die Wahrscheinlichkeit für das Herausfallen der Kanülen beim Herunterfallen des Sicherheitsbehälters erhöhen. Die Trennung von Entfernvorrichtung und Öffnung aufgrund der erfindungsgemäßen Führungsvorrichtung hat dagegen den Vorteil, daß die Öffnung sogar so klein gewählt werden kann, daß die Kanüle gerade durch die Öffnung paßt. An der Entfernvorrichtung kann jedoch beliebig viel Platz zum Hantieren mit der Spritze zur Verfügung gestellt werden, da aufgrund der Führungsvorrichtung eine Trennung der Aktionen Entfernen und Einführen in den Sicherheitsbehälter verwirklicht ist.

Der definierte Winkelbereich zur Einführung der spitzen oder scharfen Gegenstände macht auch das Herausfallen der spitzen oder scharfen Gegenstände unwahrscheinlich, denn je nach Größe des definierten Winkelbereichs ist nicht zu erwarten, daß sich beim zufälligen Umfallen des Sicherheitsbehälters die Gegenstände erstens in Richtung auf die Öffnung zu bewegen und weiter die exakte Richtung einnehmen, in der sie durch die Öffnung passen. Deshalb ist auch nach Entsorgung kein Deckel nötig, was die Arbeitszeit für den Entsorgungsvorgang drastisch reduziert, da das Entfernen und Aufsetzen, eventuell durch Verschrauben, die Zeit für das Spritzen erhöht. Vorteilhafterweise wird deshalb das Entsorgen von Spritzen über den erfindungsgemäßen Sicherheitsbehälter von dem medizinischen Personal leichter angenommen werden, und diesen auch unter Zeitdruck benutzen, beispielsweise bei einer kritischen Operation, bei der nur wenig Zeit zur Verfügung steht. Dieser psychologische Aspekt macht den erfindungsgemäßen Sicherheitsbehälter bedeutend sicherer als ähnliche Geräte nach dem Stand der Technik.

Der Sicherheitsbehälter ist vorteilhafterweise sehr einfach aufgebaut und läßt sich daher leicht herstellen, wie später noch ausführlicher dargestellt wird. Der einfache Aufbau ist vor allem darauf zurückzuführen, daß beweglichen Teile vermieden werden können.

Gemäß einer bevorzugten Weiterbildung ist die Öffnung kreisförmig und die Führungseinrichtung weist eine sich zur Öffnung erstreckende Kegelfläche auf.

Diese Ausgestaltung erleichtert ebenfalls die einfache Formgebung. Der gesamte Sicherheitsbehälter kann beispielsweise zylindrisch ausgebildet werden, wodurch am Rand eine große Fläche für verschiedene Entfernvorrichtungen zur Verfügung steht. Entsprechende Kegelflächen als Führungsvorrichtungen führen dann über Entfernvorrichtungen abgetrennte Gegenstände gleichermaßen durch die Öffnung. Wenn sogar für alle Entfernvorrichtungen die gleiche Kegelfläche zur Verfügung gestellt wird, ergibt sich die besonders einfache Form eines Sicherheitsbehälters mit einem nach innen verlaufenden, durch die Kegelfläche sich ergebenden Trichter. Der durch die Kegelfläche gebildete Trichter verbietet außerdem das Herausfallen der Gegenstände, wenn die Öffnung in Richtung der Schwerkraftwirkung gekehrt wird, da beim Umdrehen des Sicherheitsbehälters sein Inhalt an der Wand entlanggleitet und zwischen innerer Kegelfläche und Wand im Innenraum festgehalten wird.

Unbeabsichtigtes Umstoßen des Sicherheitsbehälters ist gemäß einer bevorzugten Weiterbildung dann vermeidbar, wenn mindestens ein Teilbereich der Wand so ausgebildet ist, daß der Sicherheitsbehälter mittels einer Befestigungseinrichtung an einem medizinischen Arbeitsplatz, einer Schale oder einem Spritzentablett so befestigbar ist, daß die Öffnung nach oben zeigt.

Bei dem angesprochenen Beispiel mit einer zylindrischen Wand ist diese Formgebung schon berücksichtigt. Die Befestigungseinrichtung kann dann ebenfalls ein am Arbeitsplatz befestigter Zylinder oder Ring sein, in den der Sicherheitsbehälter einfach eingeführt wird.

Eine Befestigung an einer Schale oder einem Spritzentablett gibt ebenfalls eine genügenden Sicherheit gegen das Umfällen. Sie hat aber insbesondere den Vorteil, daß das Krankenhauspersonal beim Transport der Spritzen von Patient zu Patient, den Sicherheitsbehälter immer mit sich führt und dadurch zur Hand hat. Außerdem verbleibt der Sicherheitsbehälter dann nicht beim Patienten, was beispielsweise bei Kindern sichergestellt sein muß, damit diese aus Langeweile und Spieltrieb nicht versuchen können, an die Spitzen oder scharfen Gegenstände im Innern des Sicherheitsbehälters heranzukommen.

Die Eigenschaft des erfindungsgemäßen Sicherheitsbehälters, daß die Gegenstände im Innenraum den Sicherheitsbehälter nur schwer verlassen können, macht ihn besonders als Einwegbehälter geeignet, wobei nach Füllen der gesamte Sicherheitsbehälter zusammen mit seinem Inhalt entsorgt wird. Selbst beim Weg vom Arbeitsplatz zur Müllentsorgung ist nicht zu befürchten, daß die scharfen oder spitzen Gegenstände herausfallen, es wird also auch bei späteren Arbeitsgängen eine Verletzungsgefahr vermieden.

Man kann den Sicherheitsbehälter jedoch auch als Mehrwegbehälter ausgestalten, wenn beispielsweise im Boden eine verschließbare Öffnung vorgesehen wird. Dann können die Gegenstände durch den Boden einem Sammelbehälter oder direkt dem Müll zugeführt werden. Es ist bei solchen Ausbildungen allgemein aber nicht gewährleistet, daß das scharfe oder spitze Füllgut mit der den Verschluß am Boden öffnenden Hand in Berührung kommt. Deshalb wäre auch zum Entleeren eine Einhandbetätigung wünschenswert.

Gemäß einer Weiterbildung wird dies dadurch erreicht, daß eine sich von der Innenseite des Sicherheitsbehälters eine zweite zur Öffnung erstreckende Führungseinrichtung vorgesehen wird, welche die Gegenstände beim Halten des Sicherheitsbehälters mit der Öffnung in Schwerkraftrichtung, durch die Öffnung führt.

Bei dieser Ausgestaltung wird der Sicherheitsbehälter am Arbeitsplatz, einer Spritzenschale oder einem Spritzentablett in einer Halterung lösbar befestigt, da nun die Gefahr des Herausfallens spitzer oder scharfer Gegenstände bei einem unbeabsichtigten Umstoßens geringfügig erhöht ist. Das gilt allerdings nur, wenn der Sicherheitsbehälter eine entsprechende Position nach dem Fallen einnimmt. Die zweite Führungseinrichtung kann zur Verringerung dieses Risikos auch so ausgeführt werden, daß nur von einem Teilbereich des Umfangs der Wand aus eine Entleerung möglich ist.

Die genannte Gefahr ist aber nicht gegeben, wenn die Sicherheitsbehälter gemäß der Weiterbildung in einem System, bei dem mehrere Sicherheitsbehälter an Arbeitsplätzen lösbar befestigt sind und beispielsweise einmal am Tag beim Saubermachen in einen größeren Sammelbehälter ohne zweite Führungseinrichtung und ohne Entfernvorrichtungen umgefüllt werden, der die spitzen oder scharfen Gegenstände von mehreren Arbeitsplätzen aufnimmt und der dann der Müllentsorgung zugeführt wird. Das Nehmen aus der Befestigung und das Auskippen in den Sammelbehälter wird natürlicherweise einhändig durchgeführt.

Bei eine bevorzugten Weiterbildung ist die zweite Führungseinrichtung ebenfalls als Kegelfläche ausgebildet und weist damit die gleichen Vorzüge einer einfachen Form auf wie bei der ersten Führungseinrichtung schon ausgeführt wurde.

Da die Erfindung besonders kostengünstig ausführbar sein soll, damit der Sicherheitsbehälter geeigneterweise als Einwegbehälter ausgebildet werden kann, sind auch besonders einfach herzustellende Formen für die Entfernvorrichtung vorzusehen. Gemäß einer vorzugsweisen Weiterbildung enthält die Entfernvorrichtung mindestens eine Fläche mit einem Schlitz, der sich bis zu einer Seite der Fläche erstreckt und eine zum Abstreifen der Gegenstände, insbesondere von Kanülen, geeignete Breite hat.

Die Spritze wird bei dieser Weiterbildung von der Seite der Fläche in den Schlitz eingeführt, so daß die Kanüle unter dem Schlitz liegt. Durch Hochziehen der Spritze greifen dann die Ränder des Schlitzes an der Kanüle an, so daß die Kanüle abgestreift wird und dann über die Führungseinrichtung in die Öffnung gleitet.

Die Breite des Schlitzes muß im allgemeinen dem Durchmesser der Spritze und der Kanüle angepaßt sein, damit ein derart einfaches Abstreifen möglich ist. Für unterschiedliche Spritzen könnte man dann Entfernvorrichtungen der genannten Art mit verschiedenen Schlitzbreiten vorsehen.

Der dadurch entstehende Aufwand wird dadurch verringert, daß sich gemäß einer vorteilhaften Weiterbildung der Schlitz von der an den Schlitz angrenzenden Seite der Fläche im weiteren Verlauf des Schlitzes bis zum Ende des Schlitzes verjüngt. Dadurch können Spritzen verschiedener Durchmesser in denselben Schlitz zum Abstreifen der Kanülen eingeführt werden. Der Aufwand für Entfernvorrichtungen und damit die Kosten für Sicherheitsbehälter können dadurch verringert werden.

Die vorgenannten Entfernvorrichtungen sind jedoch nur für das Abstreifen von Kanülen geeignet. Die Entsorgung von Skalpellen oder Nadeln von Einwegspritzen zur Verringerung des Sondermülls kann damit nicht durchgeführt werden.

Dazu dient gemäß einer vorzugsweisen Weiterbildung eine Brecheinrichtung, die zwei voneinander beabstandete gegenüberliegende Backen enthält, so daß der scharfe Teil eines Skalpells zwischen die Backen schiebbar und durch Bewegen eines Griffes am Skalpell abbrechbar ist.

Bei dieser bevorzugten Weiterbildung ist auch für die Brecheinrichtung eine einfache Formgebung gewählt worden, die sich besonders durch eine kostengünstige Herstellung auszeichnet, weswegen sie besonders für Einwegbehälter geeignet ist.

Wie vorhergehend schon beschrieben, ist die Gefahr des Herausfallens von scharfen oder spitzen Gegenständen vor allem beim unbeabsichtigten Umstoßen eines als Mehrwegbehälter ausgebildeten Sicherheitsbehälters gegeben, wenn dieser sich nicht in einer Befestigungsvorrichtung befindet. Um dieses Restrisiko noch weiter zu minimieren ist gemäß einer vorzugsweisen Weiterbildung der Boden als Standfläche ausgebildet, so daß auch nach einem Lösen von einer Befestigungsvorrichtung eine stabile Lage ermöglicht wird.

Die Sicherheit wird weiter durch einen aufsetzbaren Deckel erhöht, der mit Hilfe eines Gewindes oder eines Bajonettverschlusses befestigt werden kann.

Eine besonders einfache Befestigung ist gemäß einer vorzugsweisen Weiterbildung durch ein elastisches Band gegeben, welches an gegenüberliegenden Seiten bezüglich der Öffnung an der Wand befestigt ist und nach Aufsetzen des Deckels zur Befestigung über diesen gezogen werden kann. Diese Weiterbildung zeichnet sich nicht nur durch eine kostengünstige Herstellungsmöglichkeit aus, sie ermöglicht dem medizinischen Personal auch eine einfache und schnelle Befestigung, so daß in der Praxis von einer ordnungsgemäßen Befestigung selbst unter Zeitdruck Gebrauch gemacht werden wird, was beispielsweise bei einem zeitaufwendigeren Drehverschluß nicht immer erwartet werden kann.

Die Entsorgung der spitzen oder scharfen Gegenstände als Sondermüll bringt vor allem bei kleineren Artztpraxen Schwierigkeiten mit sich, da wegen der geringfügigen Mengen von Sondermüll, oftmals kein eingefahrenes System zur Entsorgung vorhanden ist. Deshalb sieht eine Weiterbildung der Erfindung einen Ofen innerhalb des Sicherheitsbehälters vor. Dieser Ofen kann dazu benutzt werden, die Spritzen, Skalpelle oder Nadeln zu sterilisieren. Wenn mit dem Ofen auch Temperaturen über 1600°C erreichbar sind, können die Gegenstände sogar eingeschmolzen werden, wodurch das Müllvolumen drastisch verringert werden kann.

Auch bei der kommunalen Beseitigung von klinischem Sondermüll wird eingeschmolzen. Deshalb ist es auch möglich, daß in Zukunft der aus dem Temperaturprozeß stammende Abfall aufgrund neuerer Bestimmungen gar nicht mehr als Sondermüll behandelt werden muß, was einen großen Vorteil für die Beseitigung der Abfälle vor allem in kleinen Artztpraxen darstellen wird. Da die Entsorgung so teilweise schon in der Arztpraxis durchgeführt wird, ist auch zu erwarten, daß das medizinische Personal den normalen Haushaltsmüll nicht leichtfertig mit infektiösem Material belasten wird, was vor allem den Schutz der Öffentlichkeit vor gefährlichen Krankheitskeimen erhöht.

Der Ofen kann in verschiedenster Weise ausgestaltet sein. Besonders vorteilhaft sind Induktionsöfen und Widerstandsheizungen mit Heizelementen aus Kanthal, Lichtbogenöfen erzeugen zwar auch hohe Temperaturen, sind aber im allgemeinen zu teuer für die Verwertung bei Sicherheitsbehältern der genannten Art.

Wenn die Heizelemente aus Kanthal sind, läßt sich ein kostengünstiges Heizen mit einfachen Mitteln erreichen. Kanthal widersteht selbst hohen Temperaturen und wird deshalb auch bei der Titanverarbeitung eingesetzt, wo ähnlich hohe Temperaturen benötigt werden. Außerdem ist die Bauweise von solchen Titanverarbeitungsöfen gut bekannt, so daß der Fachmann Konstruktionen aus diesem Bereich für Sicherheitsbehälter der dargestellten Art übernehmen kann.

Bei solchen Öfen würden aber auch giftige Gase entstehen, falls die abgestreiften Kanülen auch ein Kunststoffteil enthielten. Der Anfall dieser Gase kann durch Verwendung induktiv geheizter Öfen verringert werden, da eine induktive Heizung nur von leitenden Materialien möglich ist. Der Anfall von Verbrennungsgasen aus den Kunststoffteilen wird aber auch dann nicht vollständig unterbunden, da auch ein Wärmetransport zu den Kunststoffteilen bei den genannten hohen Temperaturen innerhalb des Ofens nicht vernachlässigbar ist.

Für die möglichen entstehenden Gase ist gemäß einer bevorzugten Weiterbildung eine Vorrichtung zum Absaugen vorgesehen. Diese kann in einem Flansch am Sicherheitsbehälter bestehen, von dem aus, nach Aufsetzen eines Deckels auf den Sicherheitsbehälter, Gase zu einem Abzug geführt werden, wobei zur effektiven Förderung der Gase auch eine Pumpe vorgesehen werden kann. Start eines zusätzlichen Flansches kann auch ein Deckel mit einer Absaugvorrichtung vorgesehen werden, so daß die Gase durch die zum Einführen der spitzen oder scharfen Gegenstände verwendete Öffnung in den Abzug entweichen. Die angesprochene Möglichkeit ist wegen der einfachen Ausgestaltung vorteilhaft, die sich daraus ergibt, daß nur ein etwas anderer Deckel als einer von denen, die vorhergehend in Verbindung mit Mehrwegsicherheitsbehältern beschrieben wurden, verwendet wird.

Gemäß einer bevorzugten Weiterbildung der Erfindung ist zwischen den Heizelementen des Ofens und der Wand eine Wärmeisolierung vorgesehen. Diese verringert nicht nur die Heizleistung, da ein kleinerer Wärmestrom durch die Sicherheitsbehälterwand abfließt, sondern verhindert auch die Verbrennungsgefahr, falls bei beheiztem Ofen das medizinische Personal in Berührung mit der Wand des Sicherheitsbehälters kommt.

Bei allen vorgenannten Möglichkeiten für einen erfindungsgemäßen Sicherheitsbehälter wurde schon der Kostenpunkt angesprochen. Der Sicherheitsbehälter sollte wegen des hohen Bedarfs für möglichst viele Arbeitsplätze im medizinischen Bereich als Massenartikel fertigbar sein. Das ist bei allen genannten Formen und Weiterbildungen, mit Ausnahme den Weiterbildungen zur Integration eines Ofens, durch ähnliche Fertigungstechniken wie bei Getränke- oder Konservendosen möglich, wenn der Sicherheitsbehälter gemäß einer bevorzugten Weiterbildung aus vorgefertigten Teilen für die Deckfläche, die Bodenfläche und die Wand unter Befestigung der vorgefertigten Teile durch Löten oder Klemmen zusammengefügt ist. Die Teile können aus Blech ausgestanzt sein. Von der Herstellung, beispielsweise einer Bierdose, unterscheidet sich ein Sicherheitsbehälter der vorgenannten zylindrischen Art mit kegelförmigen Trichter nur durch einen Formschritt für den Deckel und dadurch, daß statt des Griffes zur Öffnung die vorbeschriebenen, aus Blechen in einfache Form gebogenen Entfernvorrichtungen angelötet werden. Der beispielhafte Vergleich mit Bierdosen zeigt, daß sich die Preise für die erfindungsgemäßen Sicherheitsbehältern bei Massenfertigung auf wenige Pfennige reduzieren lassen.

Gleichfalls lassen sich die Sicherheitsbehälter auch kostengünstig aus Kunststoffteilen durch Spritzguß oder Tiefziehen fertigen. Dafür ist gemäß einer vorzugsweisen Weiterbildung vorgesehen, daß die Wand mit der Deckfläche oder die Wand mit der Bodenfläche einstückig als Kunststoffteil hergestellt ist, an dem ein die Bodenfläche beziehungsweise ein die Deckfläche enthaltendes Teil befestigt ist.

Es werden nur zwei Werkzeuge für derartige Kunststoffbehälter benötigt, die zudem einfach geformt sind, wenn eine der vorbeschriebenen Ausführungsformen für den Sicherheitsbehälter verwendet wird. Damit reduzieren sich die Kosten eines derartigen Sicherheitsbehälters bei Massenfertigung im wesentlichen auf die Kosten für das Kunststoffmaterial.

In Zusammenhang mit den verschiedenen Weiterbildungen wurde schon Arbeitsverfahren für den Umgang mit den Sicherheitsbehältern beschrieben. Allgemein ist ein erfindungsgemäßes Arbeitsverfahren zum Arbeitsschutz im klinischen Bereich, welches einen Schritt zur Behandlung eines Patienten an einem Arbeitsplatz unter Verwendung spitzer oder scharfer, an einem Halter angeordneter Arbeitsgegenstände enthält, dadurch gekennzeichnet, daß ein spitzer oder scharfer Gegenstand nach Gebrauch an einer am Arbeitsplatz, Spritzenschale oder Spritzentablett befestigten Entfernvorrichtung einhändig entfernt wird, der Gegenstand ohne manuellen Eingriff in die Öffnung eines am Arbeitsplatz, einer Spritzenschale oder einem Spritzentablett befestigten Sicherheitsbehälters innerhalb eines vorgegebenen Winkelbereichs geführt wird, wobei die Öffnung so klein ist, daß sie nur durch Ausrichtung innerhalb des vorgegebenen Winkelbereichs einen Durchgang der Gegenstände gestattet, und daß dieser bei gefülltem Sicherheitsbehälter ausgewechselt und der gefüllte entsorgt wird.

Gerade in Krankenhäusern würde die Ausrüstung jedes Sicherheitsbehälters mit einem Ofen einen nicht vertretbaren Aufwand bedeuten. Deshalb sieht eine Weiterbildung des Arbeitsverfahrens vor, daß die Entsorgung gefüllter Sicherheitsbehälter innerhalb des Klinikbereichs durch Einschmelzen in einem gemeinsamen Ofen erfolgt.

Dementsprechend betrifft die Erfindung auch ein klinisches System zur Behandlung von Patienten bei dem Arbeitsplätze gegeben sind, an denen Arbeitsgerätschaften mit spitzen oder scharfen Gegenständen, wie die Kanülen von Spritzen oder die Klingen von Skalpellen verwendet werden, bei dem Sicherheitsbehälter vorgesehen sind, die mindestens eine Entfernungsvorrichtung aufweisen, mit denen die scharfen oder spitzen Gegenstände von den Arbeitsgerätschaften einhändig entfernbar sind, und mindestens eine Führungseinrichtung, die den Gegenstand über eine Öffnung in den Sicherheitsbehälter führt, dessen Öffnung so klein ist, daß die Gegenstände nur in einem definierten Winkelbereich durch die Öffnung passen, wobei jede Führungseinrichtung so ausgelegt ist, daß sie die spitzen oder scharfen Gegenstände innerhalb des definierten Winkelbereichs in die Öffnung führt, und daß sich an mehreren der Arbeitsplätze oder an Spritzenschalen und Spritzentabletts Halter zur lösbaren Befestigung der Sicherheitsbehälter befinden.

Gemäß einer bevorzugten Weiterbildung weist das klinische System auch einen Ofen auf, in dem mit scharfen oder spitzen Gegenstände gefüllte Sicherheitsbehälter einschmelzbar sind. Dadurch wird eine Müllentsorgung schon im Klinikbereich vorgenommen, was den Abfall verringert und die Infektionsgefahr für die Öffentlichkeit außerhalb des Klinikgeländes minimiert.

Vorteilhafte Weiterbildungen ergeben sich auch aus der nachfolgenden Beschreibung von Ausführungsbeispielen in Verbindung mit den Ansprüchen und der Zeichnung. Es zeigen:
- Fig.1: ein Ausführungsbeispiel der Erfindung mit zylindrischer Wand und kegelförmiger Deckfläche;
- Fig.2: eine Entfernvorrichtung zur Entfernung von Kanülen von einer Spritze;
- Fig. 3: eine schematische Darstellung zur Erläuterung der Wirkungsweise der Entfernvorrichtung gemäß Fig. 2;
- Fig.4: eine Entfernvorrichtung für die Klingen von Skalpellen oder Nadeln von Spritzen;
- Fig. 5: ein Schnitt entlang der Längsachse eines Ausführungsbeispiels gemäß Fig.1;
- Fig. 6: eine Schnittzeichnung eines Ausführungsbeispiels für einen Mehrwegbehälter;
- Fig.7: ein Ausführungsbeispiel mit einem integrierten Ofen;
- Fig.8: Eine Schnittzeichnung für Einzelteile für eine kostengünstige Fertigung des Sicherheitsbehälters aus Kunststoff.

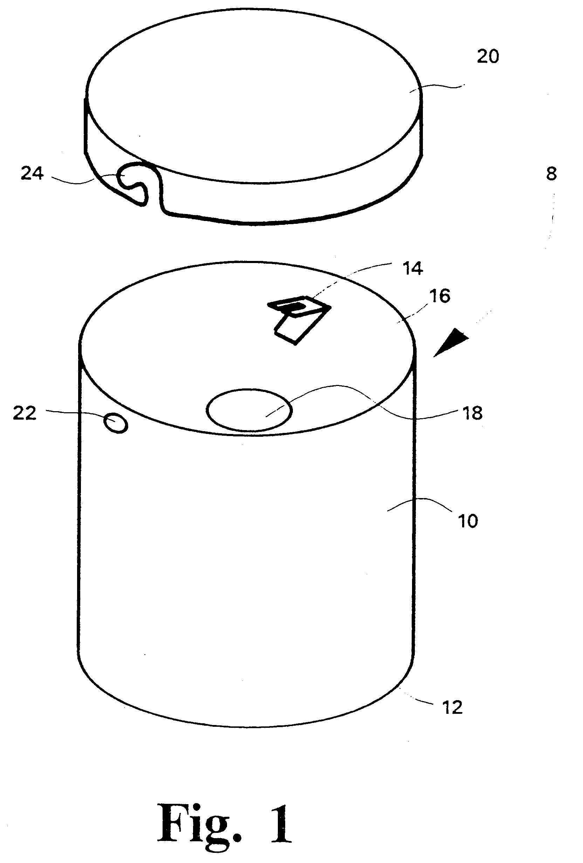

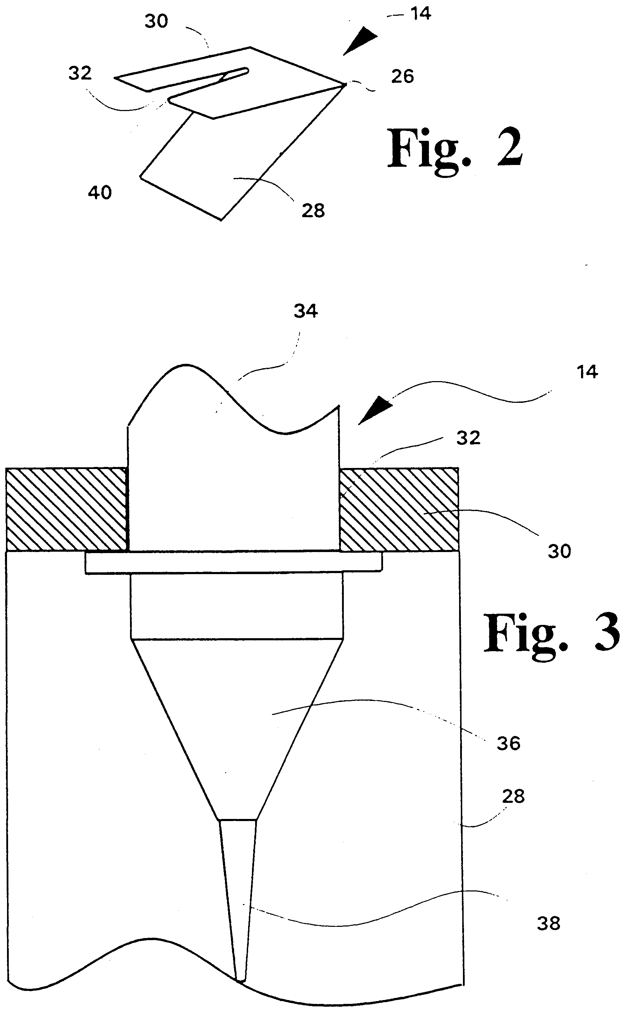

Das in Fig. 1 dargestellte Ausführungsbeispiel zeigt einen Sicherheitsbehälter 8 mit einer Wand 10. Die Wand 10 umgibt einen Innenraum, der nach unten hin durch eine Bodenfläche 12 abgeschlossen ist. An der Oberseite des Sicherheitsbehälters 8 ist eine Entfernvorrichtung 14 befestigt, an der Kanülen von Spritzen, wie in Verbindung mit Fig. 2 und Fig. 3 genauer beschrieben wird, abgestreift werden können.

Die Entfernvorrichtung 14 ist auf einer mit der Wand 10 abschließenden Deckfläche 16 angelötet. Die Deckfläche 16 ist kegelförmig ausgebildet und verläuft trichterförmig nach innen. Damit dient sie als Führungseinrichtung um an der Entfernvorrichtung abgestreifte Kanülen zu einer Öffnung 18 zu führen. Die abgestreiften Kanülen fallen dann durch die Öffnung 18 in den Innenbereich, wo sie auch bei unbeabsichtigtem Umstoßen des Sicherheitsbehälters 8 nicht mehr durch die Öffnung 18 gelangen können, wie nachfolgend anhand Fig. 5 deutlicher wird. Der Schutz vor Herausfallen ist aber zum Teil auch dadurch gegeben, daß die Öffnung 18 so klein gewählt ist, daß die Kanülen nur bei Ausrichtung innerhalb eines definierten Winkelbereichs durch die Öffnung 18 in den Innenbereich des Sicherheitsbehälters 8 gelangen können. Bei Einführen der Kanülen in die Öffnung 18 sorgt die Formgebung der kegelförmig ausgebildeten Deckfläche 16 für eine Ausrichtung der Kanülen in Richtung auf die Öffnung und in den Innenbereich aufgrund des konstruktiv festgelegten Konuswinkels des Teilkegels. Die Deckfläche 16 wirkt also nicht nur als Verschluß des Sicherheitsbehälters, sondern dient in dem Ausführungsbeispiel auch als Führungseinrichtung zum Ausrichten und Führen abgestreifter Kanülen durch die Öffnung 18.

Weiter befindet sich die Öffnung 18 zentrisch zur Wand 10 des Sicherheitsbehälters 8. Die Deckfläche 16 deckt also den Sicherheitsbehälter 8 am Rand der Wand 10 vollständig ab, wodurch beim Umfallen des Sicherheitsbehälters im Innenraum des Sicherheitsbehälters befindliche, beim Umfallen an der Wand 10 entlanggleitende Gegenstände aus dem Sicherheitsbehälter 8 nicht herausfallen könnten. Deswegen ist es wichtig, die Öffnung nicht bis zur Wand zu erstrecken. Außerdem hat die zentrische Anordnung der Öffnung 18 zur Wand 10 den Vorteil, daß am Umfang der Wand 10 auf der Deckfläche 16 noch verschiedene weitere Entfernvorrichtungen für verschiedene Zwecke angeordnet sein können, für die dann gleich günstige Bedingungen für das Führen durch die Öffnung 18 durch die als Führungseinrichtung verwendeten kegelförmig als Trichter ausgebildeten Deckfläche 16 vorliegen.

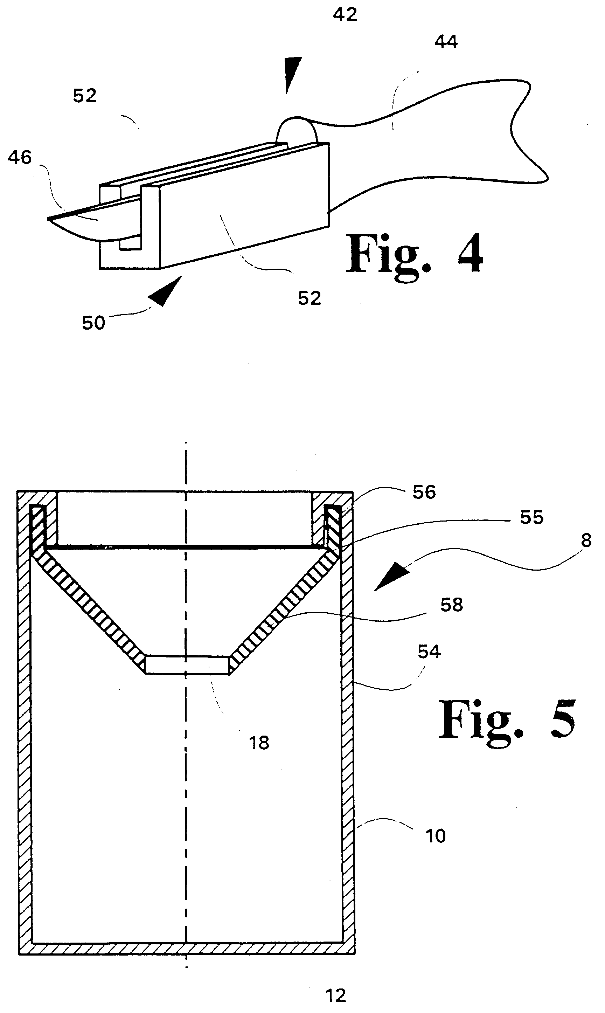

In Fig. 1 ist auch ein Deckel 20 zu sehen. Dieser Deckel ist für Einwegbehälter nicht notwendig, da bei diesen die Kanülen den Innenraum des Sicherheitsbehälters nicht unbeabsichtigt verlassen können. Mehrwegbehälter können aber bei einer Ausgestaltung gemäß Fig. 4 eine Entleerung des Sicherheitsbehälter 8 durch die Öffnung 18 vorsehen. Das unbeabsichtigte Umstoßen wird bei derartigen Sicherheitsbehältern 8 dadurch vermieden, daß dieser an der Wand 10 durch eine Befestigungsvorrichtung am medizinischen Arbeitsplatz, einer Spritzenschale oder einem Spritzentablett festgehalten wird. Die Form der Wand 10 muß dann entsprechend ausgelegt sein. Bei einer zylindrischen Wand 10, wie beim Ausführungsbeispiel, ist eine Befestigung einfach durch Einstecken, in einen am Arbeitsplatz oder einer Spritzenschale bzw. einem Spritzentablett vorgesehenen, befestigten, der Wand 10 angepaßten Zylinder möglich. Beim Herausnehmen zum Entleeren kann dann ein zusätzlicher Schutz durch Aufsetzen eines Deckels 20 über die Öffnung 18 erreicht werden. Dieser Deckel 20 sollte dann lösbar befestigt sein. Dazu kann beispielsweise ein Schraubverschluß oder ein Klemmverschluß verwendet werden. Im Ausführungsbeispiel von Fig.1 ist dagegen zur Befestigung des Deckels 20 ein Bajonettverschluß mit Eingreifteilen 22 auf der Wand 10 und entsprechenden Aussparungen 24 im Deckel 20 vorgesehen.

Ein besonders einfacher Verschluß des Deckels, der ein besonders schnelles Schließen und Öffnen des Sicherheitsbehälters 20 gestattet, besteht aus einem einfachen elastischen Band, das an gegenüberliegenden Seiten der Wand befestigt ist und nach Aufsetzen des Deckels über diesen gezogen wird. Um ein zufälliges Abrutschen des elastischen Bands vom Deckel zu vermeiden, ist es zweckmäßig, im Deckel 20 eine geeignete Aussparung oder Rille zur Aufnahme des elastischen Bands vorzusehen.

Bei Einwegbehältern, werden die Kanülen einhändig an der Entfernvorrichtung 14 abgestreift und gelangen, wie beschrieben, in den Sicherheitsbehälter 8. Dort verbleiben sie und werden mit dem Sicherheitsbehälter entsorgt.

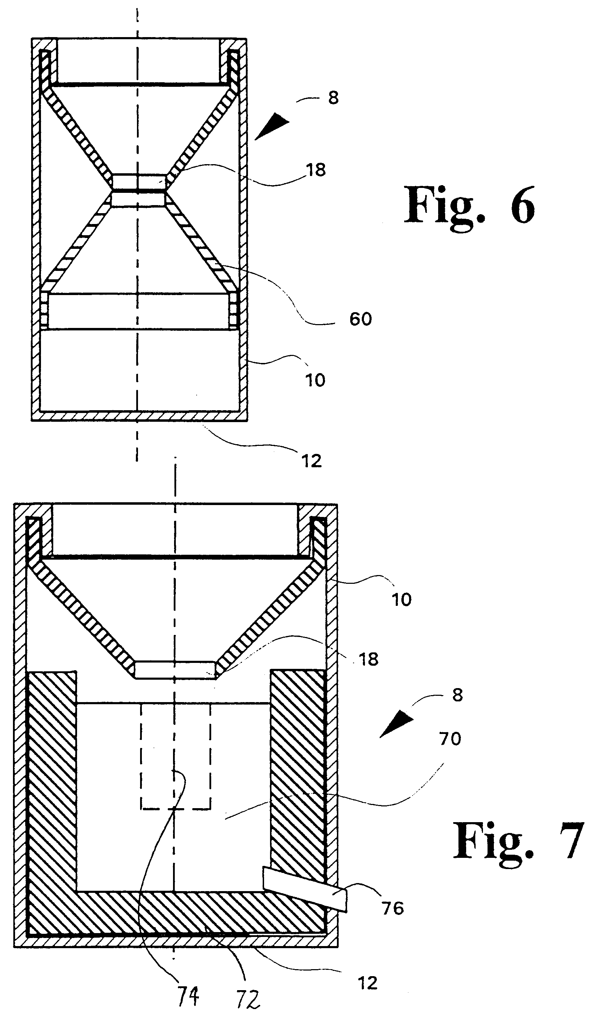

Für Mehrwegbehälter ist eine besondere Konstruktion zum Entleeren vorgesehen, die später in Verbindung mit Fig. 6 beschrieben wird. Für diese ist ein Arbeitsverfahren möglich, bei dem erst der Deckel aufgesetzt wird, der Sicherheitsbehälter zu einem anderen zu entsorgenden, als Sammelbehälter dienenden Einwegbehältern gebracht wird und dort einhändig entleert wird. Kann der Einwegbehälter zum Sammeln des Inhalts mehrerer Sicherheitsbehälter 8 zu verschiedenen Arbeitsplätzen bewegt werden, dann ist jedoch kein Deckel 20 notwendig, da er mit der gleichen Hand, mit welcher der Sicherheitsbehälter 8 aus seiner Halterung genommen wird, aufgrund der später beschriebenen Konstruktionsweise in einen Sammelbehälter ausgekippt werden kann.

Fig. 2 zeigt vergrößert die Entfernvorrichtung 14 zum Abstreifen einer Kanüle. Sie besteht im wesentlichen aus einem gefalzten Blechstück, wobei ein Falz 26 dieses in ein Befestigungsteil 28 und eine Fläche 30 unterteilt. Mit dem Befestigungsteil 28 wird die Entfernvorrichtung 14, beispielsweise durch Punkten an der Deckfläche 16, befestigt. Die dann von der Deckfläche 16 abstehende Fläche 30 ist zum Abstreifen von Kanülen an einem Schlitz 32 zum Abstreifen von Kanülen versehen. Das Abstreifen wird in Fig. 3 dargestellt.

Auf einem Spritzenkörper 34 ist eine Kanüle 36 mit daran befestigter Nadel 38 aufgesetzt. Die aus Spritzenkörper 34 und Kanüle 36 bestehende Spritze wird zur Beseitigung der Kanüle 36 von der Seite der Fläche 30, von welcher der Schlitz 32 zugänglich ist, in den Schlitz 32 geführt und zwar so, daß die Kanüle 36 sich unterhalb der Fläche 30 befindet. Die Kanüle steht im Bereich der Spritze über, so daß ein Herausziehen der Spritze nach oben gemäß Fig. 3 die Kanüle 36 von dem Spritzenkörper 34 abstreift, die dann zur Entsorgung in den Sicherheitsbehälter 8 gleiten.

Aus Fig. 3 ist auch erkennbar daß die Schlitzbreite auf die Spritzen dimensioniert werden muß, so daß für verschiedene Spritzen unterschiedliche Entfernvorrichtungen 14 mit verschiedenen Schlitzgrößen vorgesehen werden müßten, wenn der Schlitz 32 parallel verlaufende Ränder für den Abstreifvorgang aufweisen würde. Die Entfernvorrichtung 14 in Fig.2 ermöglicht jedoch das wirksame Abstreifen von Kanülen 36 bei verschiedenen Spritzendurchmessern, weil sich der Schlitz zum Falz 26 hin verjüngt. Dadurch gibt es für unterschiedliche Spritzendurchmesser innerhalb eines gewissen Bereiches immer eine Anlagefläche zum Abstreifen der Kanülen 36.

Aus Fig. 2 ist auch zu erkennen, daß der Schlitz 32 an der Seite der Fläche, an der die Spritze einführbar ist, Rundungen 40 aufweist, die das Einführen der Spritzen in den Schlitz 32 erleichtern. Das trägt zur Beschleunigung des Abstreifvorgangs für die Kanüle 36 bei.

In Fig. 4 ist ein Ausführungsbeispiel für eine Entfernvorrichtung 50 gezeigt, die geeignet ist, die Klingen von Skalpellen zu entsorgen. Das in Fig. 4 gezeigte Skalpell 42 besteht aus einem Griff 44 und einer Klinge 46. Die zum Abbrechen der Klinge 46 verwendete Entfernvorrichtung 50 besteht aus einem U-Profil, dessen mittlerer Teil zur Befestigung geeignet ist. Die Schenkel des U bilden zwei voneinander beabstandete Backen 52, zwischen welche die Klinge 46 des Skalpells 42 geschoben wird. Die Backen 52 erzeugen eine Gegenkraft bei einer Bewegung des Griffes 44 in Richtung einer Backe, so daß die Klinge abbricht und in den Innenraum des Sicherheitsbehälters 8 gleitet. Das Beispiel zeigt, daß der erfindungsgemäße Sicherheitsbehälter 8 zur Entsorgung verschiedenster mechanischer Gegenstände verwendet werden kann. Beispielsweise kann die Entfernvorrichtung 50 auch dazu verwandt werden, Nadeln von Einmalspritzen, wie sie von Diabetikern verwendet werden abzubrechen, wodurch auch in diesem medizinischen Bereich der Sondermüll verringert wird.

Fig. 5 zeigt einen Schnitt durch das Ausführungsbeispiel von Fig. 1 zur Veranschaulichung einer Fertigungsmethode für Einwegsicherheitsbehälter 8. Im wesentlich besteht der Sicherheitsbehälter 8 dabei aus einem zur Dose geformten Blech 54, in das ein Deckblech 55 mit einem zylindrischen Bereich 56 und einem kegelstimpfförmigen die Öffnung 18 am Kegelstumpfende enthaltenden Bereich 58 hineingesteckt wurde. Danach wurden die Dosenränder über den zylindrischen Bereich gefalzt, so daß das Deckblech 55 fixiert ist. Entfernvorrichtungen nach Fig. 2 oder Fig. 4 wurden anschließend auf das Deckblech aufgepunktet oder gelötet.

Neben der einfachen Herstellungsweise für einen Sicherheitsbehälter 8 verdeutlicht die Fig. 5 auch, daß der Inhalt den Sicherheitsbehälter 8 diesen beim unbeabsichtigten Umkippen nicht verlassen kann. Die Kanülen oder Skalpellklingen gleiten beim Umkippen nämlich an der Wand entlang und können sich durch keinen Kippvorgang zur Öffnung 18 bewegen, da sie selbst bei Umkehrung des Sicherheitsbehälters nicht zur Öffnung 18 gelangen. Sie werden vielmehr zwischen Deckblech 55 und dem die Wand 10 ausbildenden Blech 54 zurückgehalten.

Da sich der Inhalt eines Sicherheitsbehälters 8 nicht entfernen läßt, ist der Sicherheitsbehälter gemäß Fig.1 als Einwegbehälter geeignet, der nach Füllen als Gesamtes entsorgt wird. Deswegen ist auch eine Fertigung aus Blech sinnvoll, welches bei der Entsorgung durch Erhitzen mitschmilzt, wohingegen Kunststoffbehälter unerwünschte giftige Abgase erzeugen könnten.

Als Mehrwegbehälter kann ein Sicherheitsbehälter 8 gemäß Fig. 5 verwendet werden, wenn man beispielsweise ein Loch mit einem lösbaren Verschluß in der Bodenfläche 12 vorsehen würde, durch das der Inhalt entnommen werden kann. Dabei ist jedoch Vorsicht geboten, da das Lösen eines Verschlusses und Umschütten in einen Sammelbehälter für Sondermüll nicht unbedingt eine Einhandbetätigung erzwingt. Verletzungsgefahr ist beispielsweise dann gegeben, wenn der Sicherheitsbehälter schräg gehalten wird und der Verschluß geöffnet wird, wobei die scharfen oder spitzen Gegenstände über die den Verschluß öffnende Hand fallen können.

Um solche Arbeitsunfälle zu vermeiden, ist eine andere Bauweise gemäß Fig. 6 für Mehrwegsicherheitsbehälter geeigneter. In Fig. 6 ist ein der Fig. 5 ähnlicher Sicherheitsbehälter gezeigt, bei dem zur Öffnung 18 hin jedoch ein zweites inneres kegelförmiges Blech 60 eingesetzt wurde. Dieses Blech 60 dient als Führungseinrichtung vom Innenraum des Sicherheitsbehälters 8 nach außen.

Der Sicherheitsbehälter 8 gemäß Fig. 6 sollte in einem Halter am Arbeitsplatz, an einer Spritzenschale oder einem Spritzentablett angeordnet werden, wobei der Halter ein die Wand 10 umschließender Zylinder sein kann. Wenn der Sicherheitsbehälter 8 gemäß Fig. 6 in einen Sammelbehälter entleert wird, der im Klinikbereich von Arbeitsplatz zu Arbeitsplatz bewegt wird, ist nur eine natürliche, einhändige Bewegung erforderlich, den Sicherheitsbehälter 8 gemäß Fig. 6 aus seiner Halterung zu nehmen und in den Sammelbehälter auszuleeren. Der Sammelbehälter kann wieder wie ein Sicherheitsbehälter nach Fig. 5 oder Fig. 6 ausgebildet sein, wobei jedoch Entfernvorrichtungen unnötig sind.

Mehrwegbehälter sollten zur Abtötung von Bakterien desinfizierbar sein. Ein geeignetes Material ist daher auch Nirostastahl. Die in den Ausführungsbeispielen gezeigten Formen lassen es zu, Sicherheitsbehälter 8 mit glatten Oberflächen zu bauen, so daß es keine Ritzen gibt, in denen sich schwer zu reinigender, Infektionsträger enthaltender Schmutz festsetzen kann.

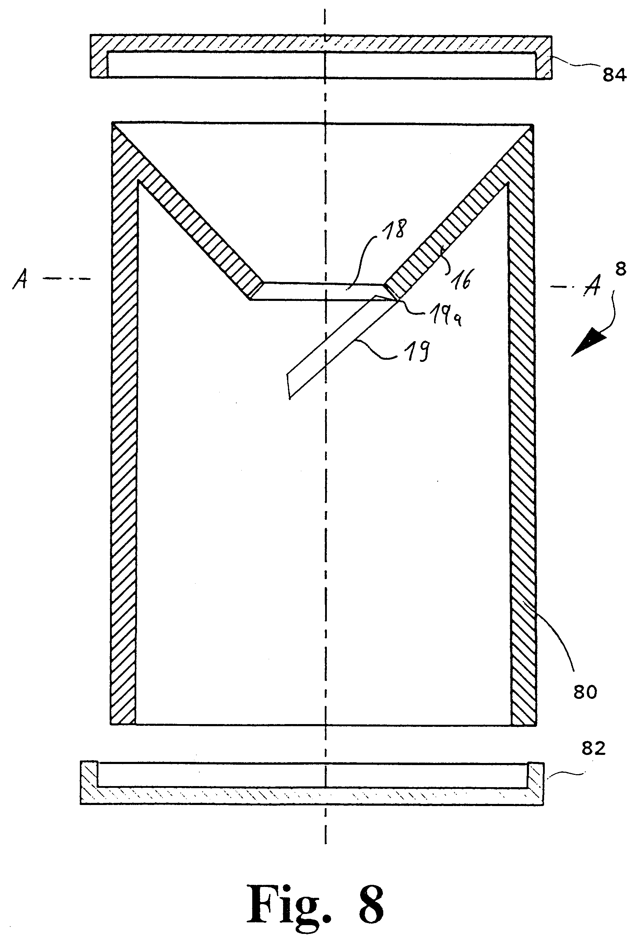

Fig. 7 zeigt eine weitere Ausführungsform für einen Sicherheitsbehälter 8, ähnlich dem von Fig. 4. Der Unterschied besteht in einem zusätzlichen Ofen 70 unterhalb der Öffnung 18. Der Ofen 70 ist durch eine Wärmeisolierung 72 von der Wand 10 des Sicherheitsbehälters 8 getrennt. Die Wärmeisolierung 70 stellt nicht nur kleine Wärmeverluste des Ofens 70 sicher, wodurch die Heizleistung des Ofens 70 gering gehalten wird, sondern hält auch die Wärme von der Wand 10 ab, damit das medizinische Personal bei der Berührung der Wand 10 keine Verbrennungen erleidet.

Der Ofen 70 ist auf eine Temperatur von 1600 ° C ausgelegt. Zur Heizung werden Kanthalwendel verwendet, die um eine Heizkammer 74 herum angeordnet sind. Die entsorgten Kanülen, Klingen o.ä. fallen durch die Öffnung 18 in die Heizkammer 74, wo sie unter Hitze geschmolzen werden. Das Schmelzgut wird anschließend aus einem Abfluß 76 entnommen. Der Schmelzvorgang verringert nicht nur das Müllvolumen, sondern tötet auch Krankheitskeime ab, so daß das nach der Wärmebehandlung entstehende Schmelzgut nicht mehr dem medizinischen Sondermüll zuzurechnen ist. Der Abfluß 76 ist seitlich, mit einer kleinen Neigung nach unten am Sicherheitsbehälter 8 angeordnet um das Schmelzgut einfach entnehmen zu können.

Ein Ausführungsbeispiel gemäß Fig. 7 eignet sich vor allem für Arztpraxen, wo die anfallenden Sondermüllmengen klein sind, und das regelmäßige Entsorgen problematisch ist. Bei Krankenhäusern wird man dagegen innerhalb eines größeren medizinischen Systems einen gemeinsamen Ofen für viele Sicherheitsbehälter zur Verfügung stellen, da dies dort eine wirtschaftlichere Entsorgung sicherstellt.

Neben Öfen, die über eine Widerstandsheizung arbeiten, wie im vorliegenden Fall, sind auch Induktionsöfen wirtschaftlich vertretbar.

Da die Kanülen auch Plastikmaterial enthalten, welches bei Wärmebehandlung Gase freisetzt, ist bei dem Ausführungsbeispiel von Fig. 7 auch vorgesehen, vor Anschaltung des Ofens 70 einen Deckel aufzusetzen, der an einem Abzug angeschlossen ist. Ein Endschalter am Sicherheitsbehälter 70 sorgt dann dafür, daß der Ofen nur dann eingeschaltet werden kann, wenn der Deckel aufgesetzt ist.

Fig. 8 zeigt ein weiteres Beispiel für den Aufbau, wenn der Sicherheitsbehälter beispielsweise aus Kunststoff gefertigt werden soll. Durch die möglichen einfachen Formen lassen sich Wand 10 und Deckelteil hier einstückig gießen, wie an dem Teil 80 in Fig. 8 verdeutlicht ist. Zum Verschließen muß dann nur noch ein Bodenteil 82 hergestellt werden, welches an das Teil 80 angeschweißt wird. Das Bodenteil hat im Beispiel von Fig. 8 die gleiche Form wie ein Deckel 84, der ebenfalls, wie vorgehend beschrieben, bei einem Sicherheitsbehälter 8 verwendet werden kann.

Die Kunststoffteile gemäß Fig.8 können durch Spritzguß oder Tiefziehen gefertigt werden. Dies erlaubt eine kostengünstige Herstellung von Sicherheitsbehältern 8. Prinzipiell lassen sich auch die in Fig. 8 nicht gezeigten Entfernvorrichtungen 14, 50 mit angießen. Wegen der beispielsweise bei der Brechvorrichtung 50 hohen wirkenden Kräfte, ist es jedoch zweckmäßiger, diese aus Stahl zu fertigen und in das Teil 80 mit einzugießen.

Die vorstehenden Beispiele zeigen Sicherheitsbehälter 8, die kostengünstig herstellbar sind, einen guten Arbeitsschutz darstellen und mit denen auch das Müllproblem verringert werden kann. Diese Sicherheitsbehälter 8 können an den Arbeitsplätzen im Klinikbereich befestigt werden. Eine besonders ergonomische Ausgestaltung sieht jedoch vor, die Sicherheitsbehälter 8 in einem Halter an einer Spritzenschale oder Spritzentablett lösbar zu befestigen, da dann das Krankenhauspersonal den Sicherheitsbehälter immer mit sich führt. Es ergibt sich dadurch auch eine zusätzliche Sicherheit da dann der Sicherheitsbehälter 8 nicht beim Patienten verbleibt, also beispielsweise bei Kindern als Patienten, auch die Gefahr eingedämmt wird, daß diese aus Spieltrieb und Langeweile den Behälter öffnen und die scharfen oder spitzen Gegenstände entnehmen.

An der dem Behalterinneren zugewandten Seite ist mit Abstand von der Öffnung 18 und einem Winkel von etwa 45° zu einer Querschnittsebene A-A der Öffnung 18 eine Ablenkfläche 19 angeordnet und an dem Trichter 16 nahe dem Rand der Öffnung 19 befestigt. Falls die Öffnung 18 durch Ausstanzen hergestellt wird, kann die Ablenkfläche 19 durch das ausgestanzte Teil selbst gebildet sein und durch einen nicht durchgestanzten Steg 19a mit dem Trichter 16 einstückig verbunden sein.

## Patentansprüche

1. Sicherheitsbehälter für von einem Halter zu lösende Gegenstände, insbesondere von einem Spritzenkörper zu lösende Kanülen, nahe dessen Behälteröffnung (18) sich eine Entfernvorrichtung (14, 50) zum Trennen des Halters von dem Gegenstand befindet, **dadurch gekennzeichnet**, daß die Entfernvorrichtung (14, 50) an der vom Behälter (8) abgelegenen Seite der Öffnung (18) angeordnet ist und eine Führungseinrichtung (16) für den gelösten Gegenstand von der Entfernvorrichtung (14, 50) in die Öffnung (18) vorgesehen ist.

2. Sicherheitsbehälter nach Anspruch 1, dadurch gekennzeichnet, daß die Führungseinrichtung die Form eines Trichters (16) hat.

3. Sicherheitsbehälter nach Anspruch 2, dadurch gekennzeichnet, daß der Trichter (16) an seinem Außenumfang mit einer Umfangswand (10) des Behälters verbunden ist.

4. Sicherheitsbehälter nach Anspruch 2 oder 3, dadurch gekennzeichnet, daß die Entfernvorrichtung (14, 50) nahe eines Randbereichs des Trichters (16) angeordnet ist.

5. Sicherheitsbehälter nach einem der Ansprüche 2 bis 4, dadurch gekennzeichnet, daß die Entfernvorrichtung (14) einen Schlitz (32) zum Abstreifen des Gegenstands von dem in den Schlitz (32) eingesetzten Halter aufweist.

6. Sicherheitsbehälter nach einem der Ansprüche 2 bis 5, dadurch gekennzeichnet, daß die Entfernvorrichtung (50) ein U-Profil zur Aufnahme einer vom Halter abzubrechenden Skalpellklinge (46) besitzt.

7. Sicherheitsbehälter nach einem der Ansprüche 1 bis 6, gekennzeichnet durch einen die Entfernvorrichtung (14, 50) und die Führungseinrichtung (16) gemeinsam überdeckenden Behälterdeckel (20).

8. Sicherheitsbehälter nach einem der vorhergehenden Ansprüche, dadurch gekennzeichnet, daß mit Abstand von der dem Behälter (8) zugewandten Seite der Öffnung (18) eine Ablenkfläche (19) angeordnet ist.

9. Sicherheitsbehälter nach Anspruch 8, dadurch gekennzeichnet, daß die Ablenkfläche (19) schräg zu einer Querschnittsebene (A-A) der Behälteröffnung (18) angeordnet ist.

10. Sicherheitsbehälter nach Anspruch 8 oder 9, dadurch gekennzeichnet, daß die Ablenkfläche (19) an der Führungseinrichtung (16) befestigt ist.
